# EUROPEAN PATENT APPLICATION

(11) **EP 4 582 786 A1**
(43) Date of publication of application: **09.07.2025**
(21) Application number: 23859326.3
(22) Date of filing: 29.08.2023
(51) Int. Cl.: G01N 1/10, G01N 33/558

(54) **TEST KIT**

(30) Priority: 29.08.2022 CN 202211065608; 31.08.2022 CN 202211066265; 07.09.2022 CN 202211091536
(71) Applicant: Leadway (HK) Limited, Sheung Wan, Hong Kong (CN)
(72) Inventor: CAI, Zhengjun, Hangzhou, Zhejiang 310030 (CN); SHANG, Tao, Hangzhou, Zhejiang 310030 (CN); TANG, Linyong, Hangzhou, Zhejiang 310030 (CN); FEI, Fengqin, Hangzhou, Zhejiang 310030 (CN); KONG, Jianxi, Hangzhou, Zhejiang 310030 (CN)
(74) Representative: Schiweck Weinzierl Koch Patentanwälte Partnerschaft mbB
(86) International application number: PCT/CN2023/115407
(87) International publication number: WO 2024/046296

(57) **Abstract**

The present invention provides a test device, including a test kit (300) and a sample collector (200). The test kit (300) includes a storage chamber (6), a bottom plate (7) with a sample injection port (3) is disposed in the storage chamber (6), an enclosure (5) is disposed on the periphery of the storage chamber (6), and a hole in communication with the interior of the test kit (300) is provided at the lower part of the enclosure or at the joint of the bottom plate (7) and the enclosure (5). The test device in the present invention can effectively prevent a liquid sample from spilling or splashing out of the sample storage chamber (6) in the process that the sample is squeezed into the test kit (6) from the sample collector, and the test kit (300) has a compact and small structure, and is very easy to carry, meeting on-site testing requirements for rapid sampling and point-of-care testing.

## Description

### Field of the Invention

The present invention relates to the technical field of rapid medical testing, in particular to a test kit and a test device for medical testing.

### Background of the Invention

The carrier of traditional disposable point-of-care testing products is basically in the form of a test strip or a test kit, for example, the test strip of a test product in the lateral flow form generally includes a bottom card, a sample pad, a label binding pad (which may also be referred to as a label pad, and generally uses glass fiber as a carrier), a test pad (which generally uses an NC film as a carrier) and an absorbent pad (which generally uses an absorbent material such as filter paper) are adhered to the bottom card in a manner of being superposed on each other in sequence from upstream to downstream, and the immunochromatographic principle is used for delivering the sample on the test strip and obtaining test results. The label pad includes labels that can be bound to an analyte, for example, latex labeled with antigens or antibodies, colloidal gold, fluorescent microspheres, etc. The test pad is generally provided with a test line and a control line. As the sample flows on the test strip, the label will be captured and gathered or not captured on the test line. The presence/absence or concentration of the analyte is determined according to the signal of the label, such as a color signal or a fluorescence signal. The control line can be used to determine whether the test strip is valid or to position an instrument when the test result is being read. As for the test kit, the test strip is placed between an upper cover and a lower plate, the upper cover is provided with a sample addition hole correspondingly above the sample pad of the test strip, and the upper cover is provided with an observation window correspondingly above the test pad.

Disposable point-of-care testing and diagnostic products for the testing of diseases or other physical conditions using body fluids such as urine, blood or other tissue fluids of a human body have been commonly used all over the world, and they can be applied in laboratories operated by professionals, or places such as home, schools, shopping malls, road checkpoints, and customs operated by non-professionals themselves.

Such traditional test products are merely simple test strips or test kits, and when sampling with a sampling stick for testing, it is necessary to put the sampling stick into a collection bottle after completion of the sampling, the sample on the sampling stick is squeezed into the collection bottle and then is dropwise added to the sample pad or the sample addition hole, as shown in Figs. 7 and 8 of U.S. Patent Application US20040237674A1, but such sample addition operation is relatively cumbersome. Chinese Patent CN200420110153.3 and Chinese Patent CN201010164579.7 improve the test kit, respectively, and simplify the steps of the sample addition operation. The sample addition hole of the test kit in Chinese Patent CN200420110153.3 protrudes out of the upper cover of the test kit to form a receiving chamber, and the internal space of the receiving chamber can receive the absorbent material on the sampling stick therein, and the liquid sample is squeezed onto the sample pad by squeezing. The sample addition port in Chinese patent CN201010164579.7 is provided with a channel, which allows passage of a sampling head, the liquid sample is squeezed out by squeezing and the sample is delivered to the sample pad via a drainage member. The sample addition holes in the two Chinese patents have some problems below, for example, the user needs to press on the sampling stick all the time to maintain the squeezing state until the liquid sample is completely squeezed, and if the sampling stick is released in the midway of the process, the liquid sample retained in the sampling stick after the loss of the squeezing force on the sampling stick will not be able to reach the sample pad, which increases the risk of insufficient sample volume; it is also possible that the sampling stick after being released falls out from the sample addition hole or the sample addition channel to contaminate the ambient environment; and the sample addition hole or the sample addition channel formed in the upper cover is blocked by the sampling stick during sample addition, the liquid sample cannot be delivered to the test strip below in time so as to be transported away but is accumulated in the sample addition region, and the squeezed sample may be squeezed by the sampling stick to spill or splash out of the sample addition region, and even splash onto the body of an operator, causing danger to the operator.

Therefore, how to provide a test device capable of achieving point-of-care testing and also preventing the sample from spilling out of the sample addition region in the sample addition process, is a technical problem urgently to be solved by the person skilled in the art at present.

### Summary of the Invention

One of the objectives of the present invention is to provide a test kit to solve the problems in the prior art. The test kit comprises a storage chamber, a bottom plate with a sample injection port is disposed in the storage chamber, an enclosure is disposed on the periphery of the storage chamber, and a hole in communication with the interior of the test kit is provided at the lower part of the enclosure or at the joint of the bottom plate and the enclosure.

Further, the test kit comprises an observation window for observing a test result, the enclosure comprises a front enclosure, a rear enclosure opposite to the front enclosure, and two side enclosures connecting the front and rear enclosures, wherein the front enclosure is closer to the observation window than the rear enclosure, the hole is provided at the lower part of the rear enclosure, or the hole is provided at the lower part of at least one of the side enclosures.

Further, the test kit comprises an observation window for observing a test result, the enclosure comprises a front enclosure, a rear enclosure opposite to the front enclosure, and two side enclosures connecting the front and rear enclosures, wherein the front enclosure is closer to the observation window than the rear enclosure, or the hole is provided at each of the lower part of the rear enclosure and the lower part of at least one of the side enclosures.

Further, the hole comprises a diversion groove provided at the joint of the bottom plate and the rear enclosure, a drain hole provided at the lower part of the rear enclosure, and a side drain hole provided at the lower part of at least one of the side enclosures, or the hole comprises at least one of the diversion groove, the drain hole and the side drain hole.

Further, the hole comprises the diversion groove provided at the joint of the bottom plate and the rear enclosure and the drain hole provided at the lower part of the rear enclosure, the diversion groove and the drain hole being separated by an isolation block.

Further, the hole comprises the diversion groove provided at the joint of the bottom plate and the rear enclosure and the drain hole provided at the lower part of the rear enclosure, the diversion groove and the drain hole being connected to form an L-shaped opening.

Further, the storage chamber comprises a first buckle therein.

Further, the first buckle is disposed on the bottom plate between the sample injection port and the wall of the storage chamber, and a spacing space is left between the first buckle and the wall of the storage chamber.

Further, the first buckle and the hole are located on opposite sides of the sample injection port on the bottom plate, respectively.

Further, a test strip located inside the test kit is comprised.

Further, a gap exists between the hole and the sampling pad when the sampling pad of the sample collector is located in the storage chamber.

Further, the test kit comprises an upper cover and a lower plate, the storage chamber being located on the upper cover.

Further, the lower plate is provided with a storage slot for placing a test strip, the test strip comprising a sample pad, and the lower plate further comprises a liquid storage tank located at the sample pad end of the storage slot.

Further, the storage slot within the liquid storage tank is provided with a notch.

Further, the notch is adjacent to the sample pad of the test strip.

Further, the notch divides the wall of the storage slot within the liquid storage tank into two parts.

Further, the diversion groove, the drain hole and the side drain hole are all located above the liquid storage tank, and orthographic projections of the diversion groove, the drain hole and the side drain hole are located within the liquid storage tank; and the diversion groove, the drain hole and the side drain hole are in fluid communication with the liquid storage tank, respectively.

One of the objectives of the present invention is to provide a test device to solve the problem of rapidly introducing the added liquid sample into a test kit in the testing process.

**In** order to achieve this objective, the present invention provides the following technical solution.

The present invention provides a test device, comprising a test kit and a sample collector; the test kit comprises a storage chamber, a bottom plate with a sample injection port is disposed in the storage chamber, an enclosure is disposed on the periphery of the storage chamber, and a hole in communication with the interior of the test kit is provided at the lower part of the enclosure or at the joint of the bottom plate and the enclosure; and a gap exists between the hole and the sampling pad when the sampling pad of the sample collector is located in the storage chamber.

The hole capable of exporting the liquid sample in time is disposed in storage chamber, and an appropriate gap exists between the hole and the sampling pad of the sample collector, the hole will not be blocked by the sampling pad so that the liquid flow channel is unblocked, therefore, the liquid sample squeezed out of the sampling pad can be guided out in time rapidly, thereby more effectively preventing the liquid sample from spilling or splashing out of the sample storage chamber when the liquid sample is squeezed into the test kit from the sample collector, which ensures that the liquid sample will not contaminate the environment around the testing because of spilling of the liquid sample in the test process, and will not fall onto the body of an operator because of splashing of the liquid sample, reducing the risk of harms to the body of the operator.

Further, the test kit comprises an observation window for observing a test result, the enclosure comprises a front enclosure, a rear enclosure opposite to the front enclosure, and two side enclosures connecting the front and rear enclosures, wherein the front enclosure is closer to the observation window than the rear enclosure, the hole is provided at the lower part of the rear enclosure, or the hole is provided at the lower part of at least one of the side enclosures.

Further, the test kit comprises an observation window for observing a test result, the enclosure comprises a front enclosure, a rear enclosure opposite to the front enclosure, and two side enclosures connecting the front and rear enclosures, wherein the front enclosure is closer to the observation window than the rear enclosure, or the hole is provided at each of the lower part of the rear enclosure and the lower part of at least one of the side enclosures.

Further, the hole provided at the lower part of the side enclosure is close to the rear enclosure.

Further, the hole comprises a diversion groove provided at the joint of the bottom plate and the rear enclosure, a drain hole provided at the lower part of the rear enclosure, and a side drain hole provided at the lower part of at least one of the side enclosures, or the hole comprises at least one of the diversion groove, the drain hole and the side drain hole.

Further, the hole comprises the diversion groove provided at the joint of the bottom plate and the rear enclosure and the drain hole provided at the lower part of the rear enclosure, the diversion groove and the drain hole being separated by an isolation block.

Further, the hole comprises the diversion groove provided at the joint of the bottom plate and the rear enclosure and the drain hole provided at the lower part of the rear enclosure, the diversion groove and the drain hole being connected to form an L-shaped opening.

Further, the storage chamber comprises a first buckle therein.

Further, the first buckle is disposed on the bottom plate between the sample injection port and the wall of the storage chamber, and a spacing space is left between the first buckle and the wall of the storage chamber.

Further, the first buckle comprises a bump and a clamping arm, the front end of the sample collector is provided with a recess, and when the sample collector is inserted into the storage chamber of the test kit, the bump of the first buckle is buckled into the recess of the collector.

Further, the first buckle and the hole are located on opposite sides of the sample injection port on the bottom plate, respectively.

### Beneficial effects

The test kit of the present invention is provided in the storage chamber with a hole capable of guiding out the liquid sample in time, so that the liquid collected in the storage chamber can flow to the interior of the test kit through the hole, avoiding that all the liquid flows into the test kit from the sampling port to result in a flooding phenomenon of the test strip in the test kit, which ultimately results in a failed test; moreover, the hole will not be blocked by a sampling pad, and can guide out the liquid sample squeezed out of the sampling pad in a rapid and timely manner, preventing the liquid sample from spilling or splashing out of the sample storage chamber in the process of squeezing the liquid sample into the test kit from the sample collector, thus ensuring that the liquid sample will not contaminate the environment around the test because of spilling of the liquid sample in the test process, and will not fall onto the body of an operator because of splashing of the liquid sample, reducing the risk of harms to the body of the operator.

### Brief Description of the Drawings

Fig. 1 is a schematic diagram of the state of use of a test device of the present invention, i.e., a schematic diagram at the point that a sample collector is just inserted into a test kit.
Fig. 2 is a schematic diagram of the state of use of the test device of the present invention, which shows a schematic diagram at the point that the sample collector is completely inserted into the test kit.
Fig. 3 is a stereoscopic exploded schematic diagram of the test device of the present invention.
Fig. 4 is a schematic diagram of a test strip in a lateral flow form.
Fig. 5 is a schematic diagram of a sample collector.
Fig. 6 is a schematic diagram of a first example of the test kit of the present invention, the bottom plate of a storage chamber of the test kit is provided with diversion grooves, the lower part of a rear enclosure is provided with drain holes, and the lower parts of side enclosures are provided with side drain holes.
Fig. 7 is a front view of the test kit in Fig. 6.
Fig. 8 is a sectional view of Fig. 7 in the direction of A-A.
Fig. 9 is a sectional view of Fig. 7 in the direction of B-B.
Fig. 10 is a schematic diagram of a second example of the test kit of the present invention, the bottom plate of the storage chamber of the test kit is provided with diversion grooves, and the lower part of the rear enclosure is provided with no drain hole.
Fig. 11 is a top view of the sample collector being inserted into the test kit.
Fig. 12 is a stereoscopic schematic diagram of the lower plate of the test kit (with a test strip assembled).
Fig. 13 is a top view of the lower plate of the test kit (with no test strip assembled).
Fig. 14 is a plan sketch of the inner surface of the upper cover and the inner surface of the lower plate of the test kit, with bidirectional arrows indicating that the upper cover and the lower plate can be buckled to each other.
Fig. 15 is a partially enlarged schematic diagram of the test kit shown in Fig. 10 at "N", and corresponds to the second example of the test kit of the present invention, the bottom plate of the storage chamber of the test kit is provided with diversion grooves, but both the lower part of the rear enclosure and the lower part of the side enclosure are provided with no drain hole.
Fig. 16 is a partially enlarged schematic diagram of the test kit shown in Fig. 6 at M, and corresponds to the first example of the test kit of the present invention, the bottom of the storage chamber of the test kit is provided with diversion grooves, the lower part of the rear enclosure is provided with drain holes, the lower part of the side enclosure is provided with a side drain hole, and the diversion groove, the drain hole and the side drain hole are in complete communication, with no isolation block among them.
Fig.17 is similar to Fig. 16 and corresponds to a third example of the test kit of the present invention, the bottom of the storage chamber of the test kit is provided with diversion grooves, the lower part of the rear enclosure is provided with drain holes, the diversion groove and the drain hole are in communication with each other, with no isolation block therebetween, and the lower part of the side enclosure is provided with no side drain hole.
Fig. 18 is similar to Fig. 16 and corresponds to a fourth example of the test kit of the present invention, the bottom of the storage chamber of the test kit is provided with diversion grooves, the lower part of the rear enclosure is provided with drain holes, an isolation block is disposed between the diversion grooves and the drain holes, but the lower part of the side enclosure is provided with no side drain hole.
Fig. 19 is similar to Fig. 16 and corresponds to a fifth example of the test kit of the present invention, the lower part of the rear enclosure of the test kit is provided with drain holes, and the lower part of the side enclosure is provided with a side drain hole.
Fig. 20 is similar to Fig. 16 and corresponds to a sixth example of the test kit of the present invention, the bottom of the storage chamber of the test kit is provided with diversion grooves, and the lower part of the side enclosure is provided with a side drain hole.
Fig. 21 is similar to Fig. 16 and corresponds to a seventh example of the test kit of the present invention, and the lower part of the side enclosure of the test kit is provided with a side drain hole.
Fig. 22 is similar to Fig. 16 and corresponds to an eighth example of the test kit of the present invention, and the lower part of the rear enclosure of the test kit is provided with drain holes.
Fig. 23 is a sectional schematic diagram of Fig. 11 in the direction of C-C, showing the state of the sample collector that is just inserted into the test kit.
Fig. 24 is similar to Fig. 23, but it shows the state of the sample collector in the process of being pressed into the test kit.
Fig. 25 is similar to Fig. 23, but it shows the state of the sample collector that has been fully pressed into the storage chamber.
Fig. 26 is an enlarged view of Fig. 25 at P, and corresponds to the example shown in Fig. 15.
Fig. 27 is similar to Fig. 26, and corresponds to the example shown in Fig. 16 or Fig. 17.
Fig. 28 is similar to Fig. 26, and corresponds to the example shown in Fig. 18.

### Detailed Description of the Embodiments

Definition: "lower part" in the specification of the present patent includes the "bottom" of a component and/or a part extending upwards from the "bottom" of the component by an appropriate concretion.

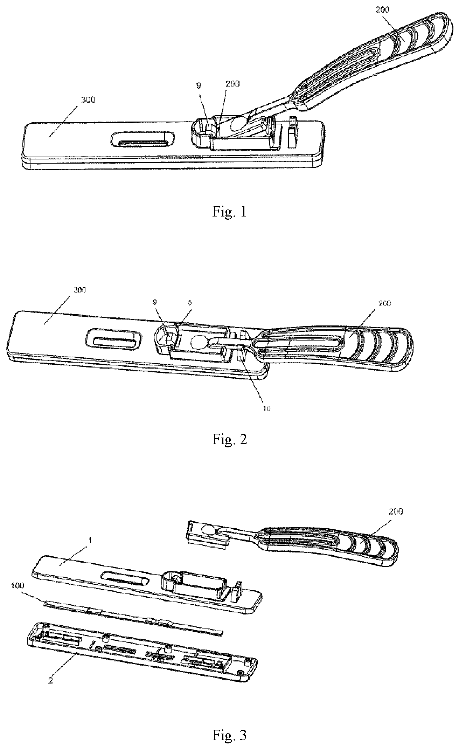

A test device, as shown in Figs. 1 to 9, includes a test kit 300 and a sample collector 200 used in cooperation with the test kit. The test kit includes an upper cover 1, a lower plate 2 and a test strip 100 located between the upper cover and the lower plate of the test kit. In one example, the upper cover 1 and the lower plate 2 are buckled to each other to hold the test strip 100 within the test kit.

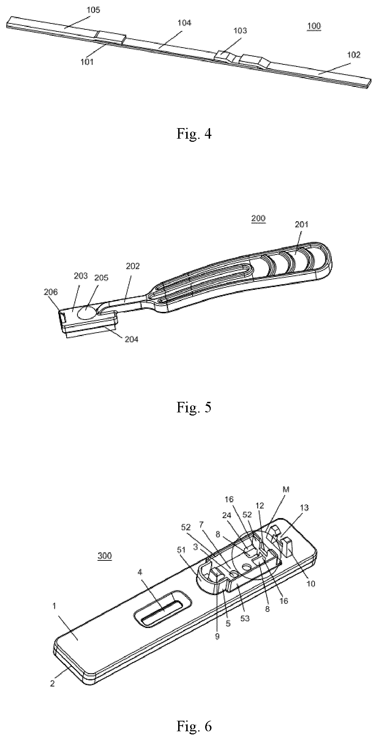

The test strip 100 as shown in Fig. 4 is a test strip in the lateral flow form, which includes a bottom card 101, and a sample pad 102, a label binding pad 103, a test pad 104 and an absorbent pad 105 are adhered to the bottom card 101 in a manner of being superposed on each other in sequence from upstream to downstream. The test strip may also be a test strip in a vertical flow form, or a test strip in other forms.

The sample collector 200 as shown in Fig. 5 includes a handle 201, a connecting portion 202, and a sampling head 203. The sampling head 203 includes a sampling pad 204. The sampling pad 204 may employ an absorbent tampon, a sponge, an absorbent fiber or a porous polymer material or the like, or an absorbent material that changes from hard to soft after absorbing water, such as polyvinyl alcohol. A sampling head 203 may also be provided with an indication region 205 located on the sampling pad, an indicator test strip is stored in the indication region, so that when the sampling pad absorbs a sufficient liquid sample, the liquid sample will come into contact with the indicator test strip, causing the indicator test strip to change from a first color to a second color, thus an operator can determine whether a sufficient sample is collected in accordance with the change in color of the indicator test strip. The handle 201 may be designed as a flat configuration with a certain curvature to facilitate holding by the operator, and the connecting portion 202 is designed in a thin-neck shape.

The upper cover 1 of the test kit 300 as shown in Fig. 6 is provided with a sample injection port 3 and an observation window 4, the sample injection port 3 is located above the sample pad 102 of the test strip, and the observation window 4 is located above the test pad 104 of the test strip. An enclosure 5 is disposed in the region around the sample injection port 3. The enclosure 5 may enclose the sample injection port 3 therein in a manner of standing on the upper surface of the upper cover and form a storage chamber 6. The enclosure 5 may also enclose the sample injection port 3 therein in a sunken manner in the way of extending downwards from the upper surface of the upper cover towards the interior of the test kit and form the storage chamber 6. The enclosure 5 may also have a part standing on the upper surface of the upper cover and have another part enclosing the sample injection port 3 therein in a manner of sinking towards the interior of the test kit and form the storage chamber 6.

The storage chamber 6 is provided with a bottom plate 7 therein, which can cooperate with the sampling pad, so that the liquid is squeezed out of the sampling pad in a manner of cooperated mutual squeezing between the bottom plate and the sampling pad, and therefore the bottom plate can also be referred to as a squeezing plate. The sample injection port 3 is formed in the bottom plate. As shown in Fig. 6, a part of the upper cover enclosing the storage chamber 6 may serve as the squeezing plate 7 that cooperates with the sampling pad, and the sample injection port 3 is located on this squeezing plate 7. A plurality of sample injection ports 3, for example, two sample injection ports spaced apart, may be disposed above the sample pad 102 of the test strip, so that on the one hand, the squeezed sample can be introduced onto the sample pad 102 of the test strip faster, and on the other hand, the strength of the squeezing plate 7 is maintained so that the squeezing plate is not prone to deformation in the squeezing process.

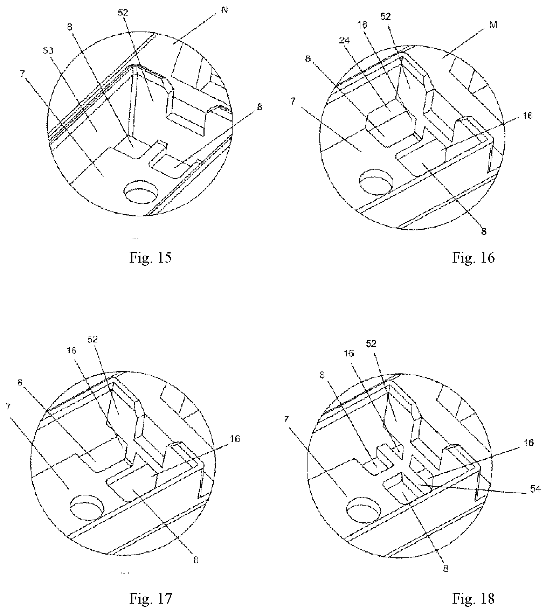

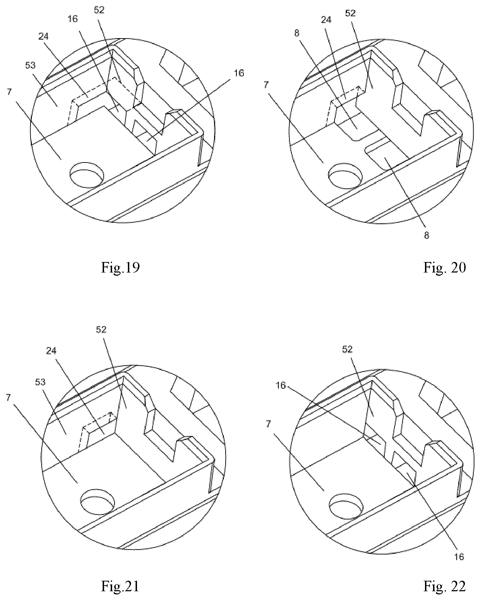

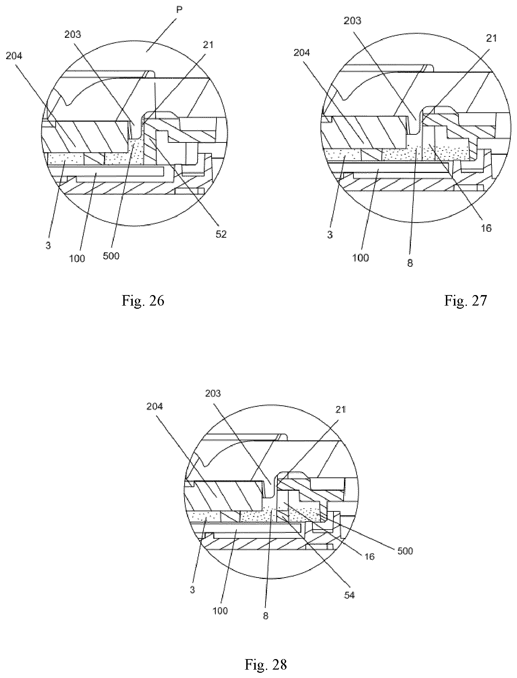

In the present invention, a hole in communication with the interior of the test kit 300 is provided at the lower part of the enclosure 5 or at the joint of the bottom plate 7 and the enclosure 5, and when the sampling pad of the sample collector 200 is located in the storage chamber 6, a gap 21 exists between the hole and the sample pad 204 (as shown in Fig. 26 to Fig. 28). The enclosure 5 includes a front enclosure 51, a rear enclosure 52, and two side enclosures 53 (as shown in Fig. 6). When the sampling pad 204 of the sample collector 200 is inserted into the storage chamber 6, the front enclosure 51 is located in the direction of the top end of the sample collector, and the rear enclosure 52 is located in the direction of the connecting portion of the sample collector (as shown in Figs. 1 and 2). The front enclosure 51 is closer to the observation window 4 than the rear enclosure 52. A hole is provided at the lower part of the rear enclosure 51 or the lower part of at least one of the side enclosures. Alternatively, a hole is provided at each of the lower part of the rear enclosure and the lower part of at least one of the side enclosure. Preferably, the hole provided at the lower part of the side enclosure 53 is close to the rear enclosure 52. More specifically, the hole includes diversion grooves 8 provided at the joint of the bottom plate 7 and the rear enclosure 52, drain holes 16 provided at the lower part of the rear enclosure 52, and a side drain hole 24 provided at the lower part of at least one of the side enclosures 53 (as shown in Fig. 16); or the hole includes at least one of the diversion groove 8, the drain hole 16, and the side drain hole 24, i.e., the hole may be one of the diversion groove 8, the drain hole 16, and the side drain hole 24 as described above, or a combination of any two of the diversion groove 8, the drain hole 16 and the side drain hole 24 as described above, or include the diversion groove 8, the drain hole 16 and the side drain hole 24 as described above at the same time (as shown in Fig. 15 to Fig. 22). Alternatively, the hole includes the diversion groove 8 provided at the joint of the bottom plate and the rear enclosure and the drain hole 16 provided at the lower part of the rear enclosure, wherein the diversion groove 8 and the drain hole 16 are separated by an isolation block 54 (as shown in Fig. 18). Still alternatively, the hole includes the diversion groove 8 provided at the joint of the bottom plate 7 and the rear enclosure 52 and the drain hole 16 provided at the lower part of the rear enclosure 52, the diversion groove 8 and the drain hole 16 being connected to form an L-shaped opening (as shown in Fig. 17).

The region within the storage chamber 6 located in the vicinity of the front enclosure 51 can be referred to as the front end of the storage chamber 6, and the region within the storage chamber 6 located in the vicinity of the rear fence 52 can be referred to as the rear end of the storage chamber 6. After the sampling pad 204 of the sample collector is located in the storage chamber 6, an appropriate gap 21 exists between the hole, located at the lower part of the enclosure 5 or at the joint of the bottom plate 7 and the enclosure 5, and the sampling pad, and the gap 21 is in communication with the hole, so that the hole will not be blocked by the sampling pad 204 and is in communication with the interior of the test kit. Thus, the present invention provides a channel for the liquid sample squeezed into the storage chamber 6 to enable the liquid sample to quickly flow to the interior of the test kit, thereby solving the technical problem in the prior art that the liquid sample is not discharged in time to result in splashing. Although Figs. 26, 27 and 28 only show that a gap 21 is located between the diversion groove 8 and/or the drain hole 16 and the sampling pad 204, the gap 21 may also be located between the side drain hole 24 and the sampling pad 204 (not shown). That is to say, the purpose of providing the gap 21 in the present invention is to prevent the diversion groove 8, the drain hole 16, or the side drain hole 24 (if the diversion groove 8, the drain hole 16, or the side drain hole 24 is present) from being blocked by the sampling pad 204. Accordingly, it is a preferred embodiment of the present invention that a gap 21 should be provided between the diversion groove 8, the drain hole 16 or the side drain hole 24 and the sampling pad 204, if the diversion groove 8, the drain hole 16 or the side drain hole 24 is present. It is appropriate that the gap 21 is sized in such a way as to enable the liquid sample to quickly flow to the interior of the test kit, thereby avoiding the generation of splashing of the liquid sample, without affecting the portability and ease of operation of the test device.

The test kit shown in Fig. 6 is provided with three diversion grooves 8 at the joint of the squeezing plate 7 and the rear enclosure 52, the diversion grooves 8 penetrate through the squeezing plate 7, therefore, the diversion grooves 8 are in communication with the interior of the test kit, so that the squeezed liquid sample can be introduced into the liquid storage tank 18 in the test kit or to the sample pad 102 of the test strip, and the sample in the liquid storage tank can be further absorbed by the sample pad of the test strip.

As shown in Figs. 6 to 9, the lower part of the rear enclosure 52 of the storage chamber 6 is provided with drain holes 16, the drain holes 16 penetrate through the squeezing plate 7, and the drain holes 16 are in communication with the interior of the test kit, so that the liquid sample flowing to the rear enclosure can enter the liquid storage tank 18 of the test kit through the drain holes 16. Both the diversion groove 8 and the drain hole 16 are located above the liquid storage tank 18, and specifically, the orthographic projections of both the diversion groove 8 and the drain hole 16 are located in the liquid storage tank. In addition, the diversion groove 8 and the drain hole 16 are in liquid communication with the liquid storage tank 18, respectively.

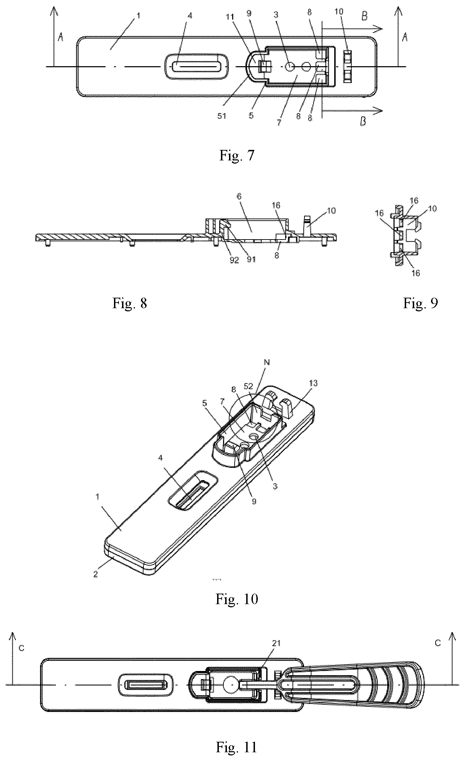

In the instance as shown in Figs. 6 and 7, the diversion groove 8 in the squeezing plate corresponds to the drain hole 16 on the lower edge of the rear enclosure 52, and the diversion groove 8 extends all the way to the drain hole 16 at the lower part of the rear enclosure. More specifically, the diversion groove 8 located in the squeezing plate and the drain hole 16 located in the rear enclosure are connected to form a right-angled opening or an L-shaped opening. Such a design may further increase the flux of the liquid sample flowing into the liquid storage tank of the test kit, avoiding that too much of the sample is retained on the squeezing plate of the storage chamber in the process of squeezing the sample collector, which causes the liquid sample to splash out of the storage chamber. The side drain hole 24 is also located above the liquid storage tank 18, specifically, the orthographic projection of the side drain hole 24 is located in the liquid storage tank, and the side drain hole and the liquid storage tank are in liquid communication.

The lower part of the side enclosure at the rear end of the storage chamber of the test kit in Fig. 6 is also provided with the side drain hole 24 to further increase the diversion capability of the rear end of the storage chamber.

For the structure of the rear end of the storage chamber of the test kit as shown in Figs. 6 and 16, the squeezing plate is provided with diversion grooves 8, the lower part of the rear enclosure is provided with drain holes 16, the lower part of the side enclosure is provided with a side drain hole 24, the diversion groove extends towards the drain hole 16 and the side drain hole 24, respectively, and intersects the drain hole 16 and the side drain hole 24, and the drain hole 16 and the side drain hole 24 are connected, so that at each of the two corners of the rear of the storage chamber, an opening consisting of the diversion groove 8, the drain hole 16 and the side drain hole 24 is formed to divert the liquid sample gathered at the rear of the storage chamber, as shown in Fig. 27.

Figs. 10 and 15 are a test kit designed in an another way of the present invention, the bottom plate of the test kit shown in Figs. 10 and 15 is provided with diversion grooves 8, the lower part of the rear enclosure 52 of the test kit is provided with no drain hole 16, and the lower part of the side enclosure 53 is also provided with no side drain hole 24. The test kit shown in Figs. 10 and 15 is adopted for sample testing; in the process of squeezing the liquid sample, the liquid, in addition to entering the test kit from the sample injection port 3, the excess liquid will flow into the test kit from the diversion grooves 8, avoiding that all the liquid flows into the test kit from the sample injection port to result in a flooding phenomenon of the test strip in the test kit, which ultimately results in a failed test; and the liquid sample can be squeezed out of the sampling pad in a rapid and timely manner, and liquid can rapidly flow to the interior of the test kit through the hole, preventing the liquid sample from spilling or splashing out of the sample storage chamber in the process of squeezing the liquid sample into the test kit from the sample collector. However, in some tests, it may also happen that the squeezed liquid splashes out of the storage chamber from a part in the vicinity of the rear enclosure. It is analyzed that the reason for this is that when the handle of the sample collector is press down by taking the first buckle as a pivot, the liquid sample stored at the front end of the sample collector is squeezed out at first, wherein a part of the sample flows into the test strip through the he sample injection port 3, and the other part of the sample that has no time to flow out of the he sample injection port flows to the rear enclosure to generate splashing liquid droplets when impacting the rear enclosure, just as high-rising sprays generated when the waves rising from a river or shore lap against the bank. It is also possible that liquid that has no time to flow into the test kit and is gathered in the vicinity of the rear enclosure is squeezed out of the rear enclosure in the mutual squeezing process of the sampling pad and the squeezing plate.

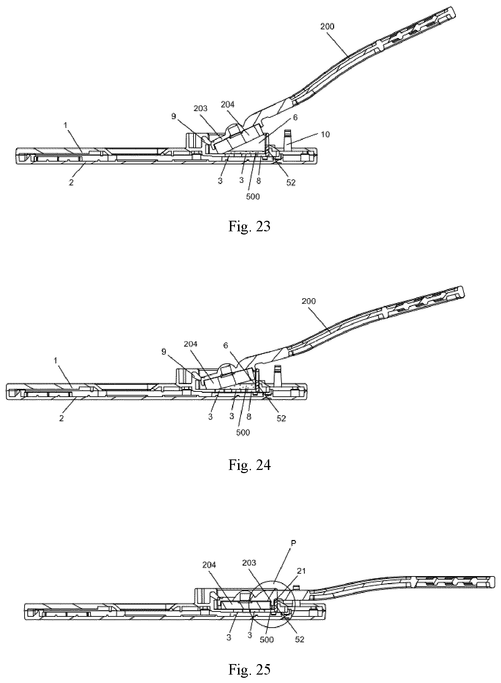

Figs. 23, 24, 25 and 26 illustrate the process of operation when the test kit shown in Figs. 10 and 15 is used for testing.

The rear end of the storage chamber of the test kit shown in Fig. 17 is provided with the diversion grooves 8 and the drain holes 16, the diversion groove 8 extending all the way to the underside of the drain hole 16. The sample collector 200 is inserted into the test kit having the structure shown in Fig. 17. As shown in Fig. 27, when the sample collector is fully pressed into the storage chamber, although most of the diversion groove 8 has been covered by the sampling pad 204, the liquid flowing to the rear end of the storage chamber can still flow into the liquid storage tank in the test kit from the drain hole 16 at the lower part of the rear enclosure, and due to the diversion effect of the drain hole, it is no longer sufficient to cause the liquid remaining in the storage chamber to flow out or to splash out of the storage chamber along the assembly gap between the sample collector and the test kit. Moreover, the liquid of the sample having no time to flow out of the sample injection port and flowing to the rear enclosure flows into the test kit from the drain hole, and it is not able to impact the rear enclosure, thereby effectively avoiding splashing generated from the impact.

The rear end of the storage chamber of the test kit shown in Fig. 18 is provided with diversion grooves 8 and drain holes 16, and an isolation block 54 is disposed between the diversion grooves 8 and the drain holes 16, and the isolation block 54 serves to improve the strength of the squeezing plate 7. As shown in Fig. 28, the sample collector 200 is inserted into the test kit having the structure shown in Fig. 18, and when the sample collector is completely pressed into the storage chamber, only a very small amount of liquid is gathered at the rear end of the storage chamber due to the diversion effect of the drain holes, and the amount of the liquid sample 500 (a plurality of "dots" are used to represent the liquid sample 500 in Fig. 28, the same throughout the article) retained at the rear end of the storage chamber is no longer sufficient to flow out or splash out of the storage chamber along the assembly gap between the sample collector and the test kit when the sampling pad is completely in contact with the squeezing plate. Even if a part of the liquid at the rear end of the storage chamber still raises the liquid level after being squeezed, the raised liquid level in the assembly gap can still flow out of the drain holes to enter into the test kit.

A set of comparative experiments is conducted using the test kit shown in Fig. 17 and the test kit shown in the prior art (i.e., a test device provided with no hole), in which the sample collector is inserted into the storage chamber of the test kit shown in Fig. 17 and the storage chamber of the existing test kit, respectively, and the situation that the liquid sample splashes out of the rear enclosure is observed. When the existing test kit is used, the squeezed liquid sample will splash out of the storage chamber, and the splashed liquid sample 500 contaminates the ambient environment. When the test kit shown in Fig. 17 is used, the squeezed liquid sample will not splash out of the storage chamber. After experimental comparison, it is found that providing the drain hole 16 in the storage chamber can effectively avoid contamination of the ambient environment as the liquid sample splashes out of the enclosure.

The opening size and height of the drain hole, the distance between the drain hole and the squeezing plate (for example, the drain hole may penetrate through the squeezing plate or may be in the squeezing plate without penetrating through the squeezing plate), and the number of the drain hole, etc. may be adjusted according to the sampling quantity of the sample collector, the capacity of the storage chamber, the sample injection rate of the sample injection port and other factors.

As verified by experiments, the test kit used in experimental group 1 is a test kit having the diversion groove, the drain hole and the side drain hole (such as the test kit shown in Fig. 16), the test kit used in experimental group 2 is test kit only having two of the diversion groove, the drain hole and the side drain hole (such as the test kit in Figs. 17, 18, 19, and 20), and the test kit used in experimental group 3 is a test kit only including one of the diversion groove, the drain hole and the side drain hole (such as the test kit shown in Figs. 15, 22, and 21), the anti-splashing effect of experimental group 1 is better than that of experimental group 2, and the anti-splashing effect of experimental group 2 is better than that of experimental group 3. Moreover, as verified by experiments, the anti-splashing effect of forming a drain hole in the enclosure at the rear end of the storage chamber is better than the anti-splashing effect of forming a diversion groove only in the bottom plate. The anti-splashing effect of forming a drain hole in the rear enclosure is better than the anti-splashing effect of forming a side drain hole on the side enclosure.

As shown in Figs. 7, 8 and 9, the upper cover 1 is also provided with a fastening device for the sample collector. One of the fastening devices is a first buckle 9 disposed in the storage chamber 6. In conjunction with Figs. 1 and 2, the sample collector 200 placed in the storage chamber 6 takes the first buckle 9 as a pivot to press down the sample collector, so that squeezing occurs between the sampling pad 204 and the squeezing plate 7 to squeeze out the liquid within the sampling pad. In the instances shown in Figs. 1 and 2, and Figs. 7 to 9, the first buckle 9 is provided with a bump 91 at the upper end (as shown in Fig. 8), and the sample collector is provided with a recess 206 at the top end (as shown in Fig. 1). When the sample collector is inserted into the storage chamber, the top end of the mounting plate 203 for the sampling pad is placed under the first buckle 9, the bump 91 of the first buckle 9 is buckled in the recess 206 of the sample collector, and then the buckled point is taken as the rotating pivot of an axis to press down the handle 201 of the sample collector, so that squeezing occurs between the sampling pad 204 and the squeezing plate 7. The manner of forming pivot fit between the first buckle and the sample collector may also be in other manner, but not limited to, those listed below, for example, the upper end of the first buckle 9 is provided as a recess and the top end of the sample collector is set as a bump. Alternatively, the first buckle is provided with an aperture, the sample collector is provided with an insertion bugle at the top end, and the bugle can be inserted into the aperture to form a pressing pivot.

The first buckle 9 is disposed at the front end of the storage chamber. For example, the first buckle 9 may be disposed on the inner wall of the front enclosure 51. For another example, in the example shown in Fig. 7, the first buckle 9 is disposed on the bottom plate between the sample injection port 3 and the front enclosure 51, and a spacing space 11 is left between the first buckle 9 and the front enclosure 51 (as shown in Fig. 7). That is, the first buckle 9 and the hole (e.g., the diversion groove 8) are located on opposite sides of the sample injection port 3 in the bottom plate. In the instance where the first buckle is a buckle having certain elasticity, the spacing space 11 leaves a retraction space for the first buckle 9. It is specifically embodied in that when the sample collector is inserted into the storage chamber 6, the front end of the collector presses against a clamping arm 92 of the first buckle 9 (as shown in Fig. 8), and the clamping arm after being pressed undergoes stressed elastic tilt towards the spacing space until the bump 91 of the first buckle is buckled in the recess 206 of the collector, and the clamping arm is restored to its original position.

In the example where the spacing space 11 is provided between the first buckle 9 and the front enclosure 51, the spacing space 11 may also play a role of cushioning. In the method that the first buckle 9 is disposed on the inner wall of the front enclosure, the top end of the sample collector 200 inserted into the storage chamber 6 is very close to the front enclosure, so that if the amount of the liquid sample squeezed out of the front end of the sampling pad is larger than the assembly gap between the top end of the collector and the front enclosure, and this part of liquid has no time to flow into the test kit, the liquid sample located within the assembly gap between the top end of the collector and the front enclosure might be squeezed out of the enclosure, thus the provided spacing space 11 increases the temporary storage space for the liquid sample, which is equivalent to a buffer region.

The present invention may also include another fastening device, which is a second buckle 10 disposed behind the rear enclosure 52. The clamping slot 13 of the second buckle 10 is on the same axis as the groove 12 of the rear enclosure. After the sample collector 200 squeezes the sampling pad completely against the squeezing plate 7 (bottom plate), the connecting portion 202 of the sample collector is buckled into the second buckle and is confined within the clamping slot 13 of the second buckle, and the sampling pad remains in a squeezed state after the operator releases the collector.

As shown in Figs. 1, 2, and 6, the rear enclosure 52 is also provided with a groove 12 for receiving the connecting portion 202 of the sample collector. When the sample collector 200 is pressed down by taking the first buckle 9 as a pivot, the connecting portion 202 of the collector can be embedded in the groove 12 of the rear enclosure, so that the sampling pad 204 of the collector 200 can be completely squeezed on the squeezing plate 7 in a flat manner, thus sufficiently squeezing every position on the sampling pad 204.

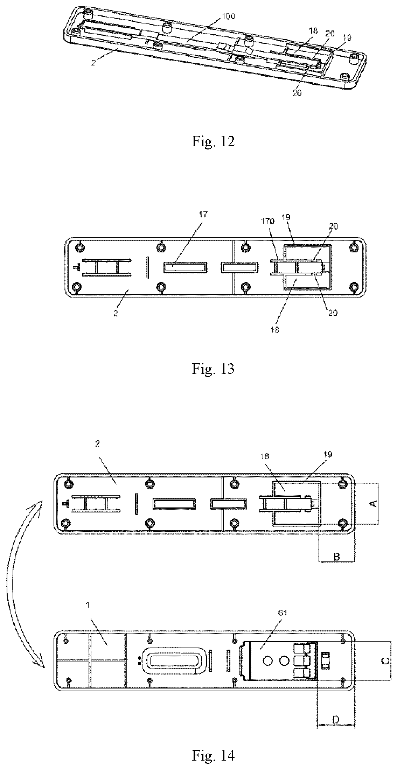

The lower plate 2 of the test kit is provided with a storage slot 17 for the test strip 100, and a liquid storage tank 18 is disposed at the sample pad end of the storage slot. In the example shown in Figs. 12 and 13, the liquid storage tank is enclosed by the wall 19 of the liquid storage tank and the wall 170 of the storage slot at the sample pad end.

One function of the liquid storage tank is to collect and temporarily store the liquid sample squeezed out of the sample collector. The sample injection port of the test kit should not be too large, as an excessively large sample injection port may cause a large amount of liquid sample to instantly reach the test pad of the test strip, causing a Flooding phenomenon of the test strip, which affects the test result. The test kit of the present invention allows a part of the liquid sample to flow to the test pad of the test strip through the sample injection port 3, and the other part of the liquid flows into the liquid storage tank of the test kit through flow-assisting holes and/or the drain holes, and the liquid sample in the liquid storage tank can continue to be slowly absorbed by the test pad of the test strip until the test dosage requirements are met.

Providing a notch 20 in the wall of the storage slot 17 within the liquid storage tank 18 (as shown in Figs. 12 and 13) is a way of diverting the liquid sample within the liquid storage tank to the test strip. The liquid sample collected within the liquid storage tank can enter the storage slot 17 through the notch 20 and be absorbed by the sample pad within the storage slot. The provision of the notch can more effectively direct the liquid within the liquid storage tank to contact the sample pad of the test strip.

As shown in Fig. 14, the width of the bottom plate at the storage chamber is smaller than the width of the liquid storage tank of the lower plate. When the upper cover and the lower plate are buckled together, the wall of the liquid storage tank encloses the bottom 61 of the storage chamber therein, which can effectively control the liquid flowing into the liquid storage tank within the liquid storage tank, and avoid that the sample not absorbed by the sample pad flows around in the test kit. Specifically as shown in Fig. 14, the transverse width of the bottom edge of the wall of the liquid storage tank of the lower plate is 13.6cm (the distance as indicated at "A" of Fig. 14), the distance from the bottom edge of the tank wall to the bottom edge of the lower plate of the test kit is 12cm (the distance as indicated at "B" of Fig. 14), the transverse width of the bottom plate of the storage chamber of the upper plate is 13cm (the distance as indicated at "C" of Fig. 14), the closest distance from the bottom plate of the storage chamber of the upper cover to the bottom edge of the upper cover of the test kit is 12.5cm (the distance as indicated at "D" of Fig. 14), and when the upper cover and the lower plate are buckled together, the wall of the liquid storage tank encloses the bottom plate of the storage chamber therein.

### Example 1 Preparation of a portable SARS-CoV-2 test device

The preparation of a portable SARS-CoV-2 test device is taken as an example.

The SARS-CoV-2 test strip 100 includes a bottom card 101, and a sample pad 102, a label binding pad 103, a test pad 104 and an absorbent pad 105 are adhered to the bottom card 101 in a manner of being superposed on each other in sequence from upstream to downstream. The label binding pad 103 is coated with an anti-SARS-CoV-2 antibody-latex label, the test line (T line) of the test pad is coated with an anti-SARS-CoV-2 antibody, and the control line (C line) is coated with goat-anti-mouse IgG.

The prepared SARS-CoV-2 test strip is put into the test kit 300 such that the sample injection port 3 of the upper cover 1 of the test kit is located above the sample pad 102 of the test strip and the observation window 4 is located above the test pad 104 of the test strip.

The sample collector 200 is encapsulated in a sealing bag to form a complete test device with the prepared test kit. The sampling pad of the sample collector 200 absorbs water and swells upon contact with saliva.

### Example 2 Use of the test device

The use of the portable SARS-CoV-2 test device is taken as an example.

The sample collector 200 is removed from the sealing bag, and the sample collector is put into the mouth of a tester to aspirate saliva to a specified sample volume. If an indicator test strip is available, one can determine whether a sufficient volume has been collected according to the color change of the indicator test strip. If no indicator test strip is available, one can determine whether a sufficient volume has been collected according to the swelling degree of the sampling pad.

The sample collector with sufficient volume collected is inserted into the storage chamber 6 of the test kit, the first buckle 9 within the storage chamber is buckled with the groove at the front end of the collector, the sample collector 200 is pressed down by taking the first buckle 9 as the pivot, the connecting portion 202 of the collector is embedded into the groove 12 of the rear enclosure, and when the sampling pad 204 of the collector 200 is completely squeezed on the squeezing plate 7 in a flat manner, the connecting portion 202 is buckled into the second buckle and is confined within the clamping slot of the second buckle, at which point the operator can release the collector and the collector can be always held in the squeezed state.

A part of the liquid sample squeezed out of the sampling pad of the collector reaches the sample pad of the test strip through the sample injection port 3, and the other part of the liquid sample enters the liquid storage tank of the lower plate through the diversion groove 8 or the drain hole 16, and flows into the sample pad through the notch 20 of the test strip storage slot.

The liquid sample reaches the absorbent pad 105 via the sample pad 102, the label binding pad 103, and the test pad 104. If the test pad only shows a color at the position of the C line, it indicates that the sample is negative, if a color is shown at positions of both the C line and the T line, it indicates that the sample is positive and further nucleic acid analysis is required, and if no color is shown at the position of the C line, it indicates that this test is invalid.

### Example 3 Portable drug test device and use thereof

The drug test strip 100 is also referred to as a drug of abuse test strip, and "drug of abuse" (DOA) is a drug that is used for non-medical purposes (usually for psychedelic effects). Abuse of such drug can lead to physical and mental hurts as well as (in some cases) dependency, addiction, and even death. Instances of DOA include cocaine, amphetamines (e.g., black beauties, white bennies, amphetamine tablets, dextroamphetamines, dexies, beans), methamphetamines (crank, methamphetamine, crystal, speed), barbiturates (Valium^{®}, Roche Pharmaceuticals, Nutley, New Jersey), sedatives (i.e., sleeping pills), lysergic acid diethylamide (LSD), tranquilizers (downers, goofballs, barbs, blue devils, yellow jackets, ludes), tricyclic antidepressants (TCAs, e.g., promethazine, amitriptyline, and doxepin), phencyclidine (PCP), tetrahydrocannabinol (THC, pot, dope, hash, weed, etc.), and opiates (e.g., morphine, opium, cocaine, heroin, oxycodone).

A portable amphetamine (urine) colloidal gold test device is taken as an example in this example.

The portable amphetamine (urine) colloidal gold test device includes a test strip, a test kit and a sample collector. The test strip is a portable amphetamine (urine) colloidal gold test strip, including a bottom card, and a sample pad, a label binding pad, a test pad and an absorbent pad are adhered to the bottom card in a manner of being superposed on each other in sequence from upstream to downstream. The principle of binding amphetamine couplers and amphetamine that may be contained in the urine through competition of monoclonal antibodies. The label binding pad contains an anti-amphetamine monoclonal antibody (colloidal gold antibody) labeled with colloidal gold, and the T line on the test pad contains an amphetamine coupler.

During the test, the sample collector enters the urine of the tester and the urine sample on the collector is then squeezed into the test kit, where the urine sample is then chromatographed upwards on the test strip by capillary effect. If the concentration of amphetamine in the urine sample is less than 1000ng/ml, the colloidal gold antibody cannot bind to all of the amphetamine. Thus, the colloidal gold antibody will be bound by the amphetamine coupler immobilized on the test pad in the chromatographic process and a purple-red stripe will appear on the T line. If the concentration of amphetamine in the urine sample is higher than 1000ng/ml, the colloidal gold antibody binds to all of the amphetamine, and thus no purple-red stripe appears in the T-line region due to no bonding to the amphetamine coupler because of a competing reaction. The negative urine sample will show a purple-red stripe on the T line due to the lack of antibody-antigen competing reactions in the testing process. A purple-red stripe will appear at the control line C no matter whether amphetamine is present in the urine sample or not. The purple-red stripe shown in the control region (C) is the standard for determining whether a sufficient urine sample is available and whether the chromatographic process is normal, and also serves as an internal controlled standard for the reagent.

In the example where one test device is used for testing multiple drugs of abuse, a plurality of test strip storage slots are formed in the lower plate of the test kit for placing test strips for different test items.

Example 4 Portable immunofluorescence analysis (FIA) test device and use thereof
The test strip in this example takes time-resolved immunofluorescence testing based on the phosphorescence luminance technology as an example.

The test strip is used for testing IgG antibodies generated after infection by a pathogenic microorganism, with the test mode being an indirect method mode. The preparation method of the test strip includes: labeling an anti-human immunoglobulin IgG antibody with a phosphorescent material and immobilizing the labeled antibody on the label bonding pad; immobilizing the pathogenic microbial antigen to be analyzed on the test line of the test pad; and immobilizing the IgG on the control line of the test pad.

During testing, the liquid sample on the collector is squeezed into the test kit, then the collector is pulled out, the test kit containing the test strip described in this example is inserted into a fluorescence analyzer, and the test result is analyzed through the value of the intensity of phosphorescent signals of the T line and C line of the test strip. When both the test line and the control line generate a phosphorescent signal at the same time, it is a positive result, indicating that the sample contains the target substance to be tested; and when the test line does not generate a phosphorescent signal but the control line generates a phosphorescent signal, it is a negative result, indicating that the sample does not contain the target substance to be tested.

## Claims

1. A test kit, comprising a storage chamber, wherein a bottom plate with a sample injection port is disposed in the storage chamber, an enclosure is disposed on the periphery of the storage chamber, and a hole in communication with the interior of the test kit is provided at the lower part of the enclosure or at the joint of the bottom plate and the enclosure.

2. The test kit according to claim 1, wherein comprising an observation window for observing a test result, the enclosure comprising a front enclosure, a rear enclosure opposite to the front enclosure, and two side enclosures connecting the front and rear enclosures, wherein the front enclosure is closer to the observation window than the rear enclosure; and the hole is provided at the lower part of the rear enclosure, or the hole is provided at the lower part of at least one of the side enclosures, or the hole is provided at each of the lower part of the rear enclosure and the lower part of at least one of the side enclosures.

3. The test kit according to claim 2, wherein the hole comprises a diversion groove provided at the joint of the bottom plate and the rear enclosure, a drain hole provided at the lower part of the rear enclosure, and a side drain hole provided at the lower part of at least one of the side enclosures, or the hole comprises at least one of the diversion groove, the drain hole and the side drain hole.

4. The test kit according to claim 2, wherein the hole comprises the diversion groove provided at the joint of the bottom plate and the rear enclosure and the drain hole provided at the lower part of the rear enclosure, the diversion groove and the drain hole being separated by an isolation block.

5. The test kit according to claim 2, wherein the hole comprises the diversion groove provided at the joint of the bottom plate and the rear enclosure and the drain hole provided at the lower part of the rear enclosure, the diversion groove and the drain hole being connected to form an L-shaped opening.

6. The test kit according to claim 1, wherein the storage chamber comprises a first buckle therein.

7. The test kit according to claim 6, wherein the first buckle is disposed on the bottom plate between the sample injection port and the wall of the storage chamber, and a spacing space is left between the first buckle and the wall of the storage chamber.

8. The test kit according to claim 6, wherein the first buckle and the hole are located on opposite sides of the sample injection port in the bottom plate, respectively.

9. The test kit according to claim 1, wherein a gap exists between the hole and the sampling pad when the sampling pad of the sample collector is located in the storage chamber.

10. The test kit according to claim 1, wherein the test kit comprises an upper cover and a lower plate, the storage chamber being located on the upper cover.

11. The test kit according to claim 10, wherein the lower plate is provided with a storage slot for placing a test strip, the test strip comprising a sample pad, and the lower plate further comprises a liquid storage tank located at the sample pad end of the storage slot.

12. The test kit according to claim 11, wherein the storage slot within the liquid storage tank is provided with a notch.

13. The test kit according to claim 12, wherein a hole provided at the lower part of the enclosure or at the joint of the bottom plate and the enclosure is located above the liquid storage tank and in fluid communication with the liquid storage tank, and the orthographic projection of the hole is located within the liquid storage tank.

14. The test kit according to claim 13, wherein the hole is selected from the diversion groove, the drain hole, the side drain hole or a combination thereof.

15. A test device, comprising a test kit and a sample collector, wherein the sample collector comprises a storage chamber, a bottom plate with a sample injection port is disposed in the storage chamber, an enclosure is disposed on the periphery of the storage chamber, and a hole in communication with the interior of the test kit is provided at the lower part of the enclosure or at the joint of the bottom plate and the enclosure.

16. The test device according to claim 15, wherein the test kit comprises an observation window for observing a test result, the enclosure comprises a front enclosure, a rear enclosure opposite to the front enclosure, and two side enclosures connecting the front and rear enclosures, wherein the front enclosure is closer to the observation window than the rear enclosure; and the hole is provided at the lower part of the rear enclosure, or the hole is provided at the lower part of at least one side enclosure, or the hole is provided at each of the lower part of the rear enclosure and the lower part of at least one of the side enclosures.

17. The test device according to claim 15, wherein the test kit comprises a test strip, an upper cover and a lower plate, the storage chamber is formed by the enclosure standing on the upper surface of the upper cover, the lower plate is provided with a storage slot, and the storage slot is provided with a liquid storage tank at the sample pad end of the test strip.

18. The test device according to claim 17, wherein the wall of the storage slot is provided with a notch, through which the liquid sample in the liquid storage tank can enter the sample pad in the storage slot.

19. The test device according to claim 17, wherein a hole provided at the lower part of the enclosure or at the joint of the bottom plate and the enclosure is located above the liquid storage tank and in liquid communication with the liquid storage tank, and the orthographic projection of the hole is located in the liquid storage tank.

20. The test device according to claim 15, wherein a gap exists between the hole and the sampling pad when the sampling pad of the sample collector is located in the storage chamber.
